# EUROPEAN PATENT APPLICATION

(11) **EP 1 225 232 A2**
(43) Date of publication of application: **24.07.2002**
(21) Application number: 02001232.4
(22) Date of filing: 17.01.2002
(51) Int. Cl.: C12Q 1/68

(54) **Detection of mutations in a gene encoding lkappaB kinase-complex-associated protein to diagnose familial dysautonomia**

(30) Priority: 17.01.2001 US 262284 P
(71) Applicant: Rubin, Berish Y., Monsey, New York (US); Anderson, Silvia L., Dumont, New Jersey (US)
(72) Inventor: Rubin, Berish Y., Monsey, New York (US); Anderson, Silvia L., Dumont, New Jersey (US)
(74) Representative: Helbing, Jörg, Dr.

(57) **Abstract**

A method for detecting the presence in a subject of a polymorphism linked to a gene associated with familial dysautonomia, said method comprising detecting a disruptive mutation in a gene of said subject encoding the IκB kinase-complex-associated protein, and, preferably, detecting a T → C change in position 6 of the donor splice site of intron 20 and/or a G → C transversion of nucleotide 2390 in exon 19 of the gene encoding the IκB kinase-complex-associated protein which is present on chromosome 9q31. Also disclosed are oligonucleotide primers useful in the detection method. This abstract is provided to comply with the rules requiring an abstract that will allow a searcher or other reader to ascertain quickly the subject matter of the technical disclosure. It is submitted with the understanding that it will not be used to interpret or limit the scope or meaning of the claims. 37 CFR § 1.72(b).

## Description

This invention relates to a method for the diagnosis of Familial Dysautonomia, and for the identification of carriers of Familial Dysautonomia, and to a mutated gene and altered proteins associated therewith.

Familial Dysautonomia (FD), also known as the Riley-Day syndrome or hereditary sensory neuropathy Type III (MIM 223900), is an autosomal recessive disorder that affects the development and survival of sensory, sympathetic and some parasympathetic neurons (*1,2*). Individuals with FD are affected with a variety of symptoms that include decreased sensitivity to pain and temperature, cardiovascular instability, recurrent pneumonias, vomiting crises, and gastrointestinal dysfunction (*1-3*). It has been suggested that these symptoms might be due to a deficiency in a neuronal growth factor pathway (*4,5*). This disorder is primarily confined to individuals of Ashkenazic Jewish descent (*6*). Based on the birth incidence of FD, the predicted carrier frequency of this defective gene (*DYS*) is approximately 1 in 30 (*7*). Haplotype analysis using various polymorphic loci has enabled the localization of FD to chromosome 9q31 and the demonstration that there is a major haplotype representing >98% of the FD chromosomes (*8*). Several additional FD haplotypes represent the remaining 2% of FD chromosomes. The defective gene, *DYS,* which has been mapped to a 0.5 cM region on chromosome 9q31, has eluded identification.

Identification of the gene and/or altered protein associated with FD would be valuable, as knowledge of such gene and/or altered protein would allow more accurate screening of the entire at-risk population to identify carriers, and potentially to reduce the incidence of new cases. Thus, genetic testing would permit the identification of carriers of a disease gene, and also the evaluation of the condition of unborn children who may be affected with the disease or carriers of the disease gene. Knowledge of the gene and/or altered protein associated with FD might also facilitate the development of effective therapeutic approaches for individuals with FD.

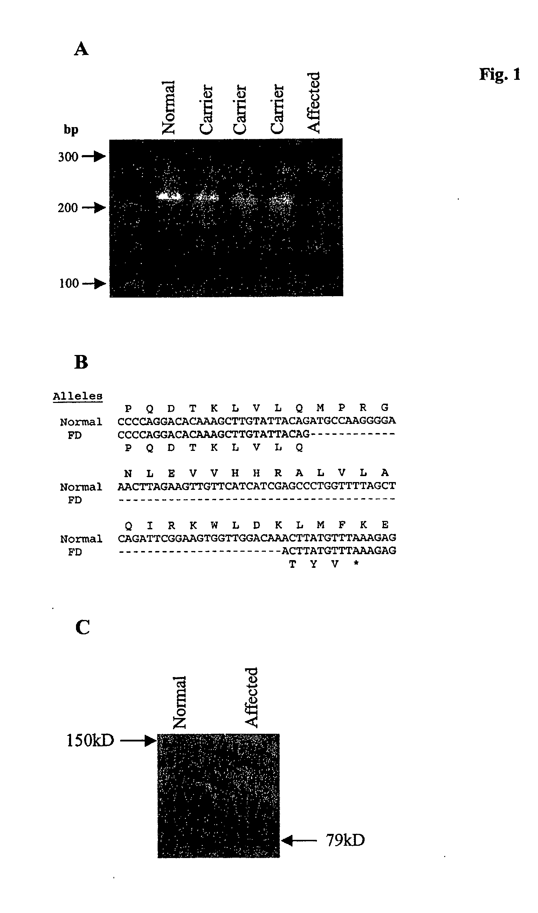

Using DNA sequence information available as a result of the Human Genome Project, we determined the location of the polymorphic markers 164D1, D9S1677 and 157A3 that have been reported to be localized to the area of the *DYS* gene (*8*) and identified several mRNAs encoded in this region. RT-PCR analysis performed on these RNAs revealed that primers to the transcript reported to encode IKAP (*9*), which generated the predicted 218 bp product from RNA isolated from a control lymphoblast cell line, generated a 144 bp PCR product from RNA derived from a lymphoblast cell line generated from an individual homozygous for the major FD haplotype. The same primers generated both the 218 and 144 bp products from RNA isolated from lymphoblast cell lines derived from individuals heterozygous for the major FD haplotype (Fig. 1A). Identical results were obtained when using RNAs from fibroblast cell lines derived from normal individuals and individuals homozygous and heterozygous for *DYS* (data not shown). Sequence analysis of the RT-PCR products revealed that the IKAP mRNA generated by the *DYS*-bearing chromosome does not contain exon 20 and that the predicted translation of this transcript generates a truncated protein with a molecular weight of approximately 79 kD (Fig. 1B). Western blot analysis using antibody to IKAP reveals the presence of a 150 kD protein in control cells and a 79 kD protein in cells derived from individuals homozygous for the major FD haplotype (Fig. 1C).

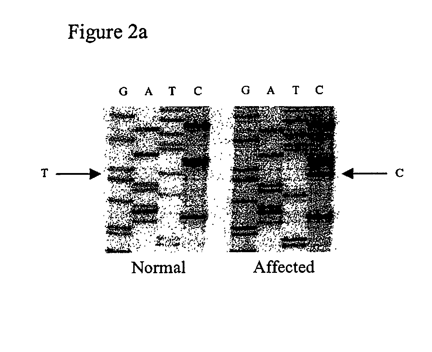

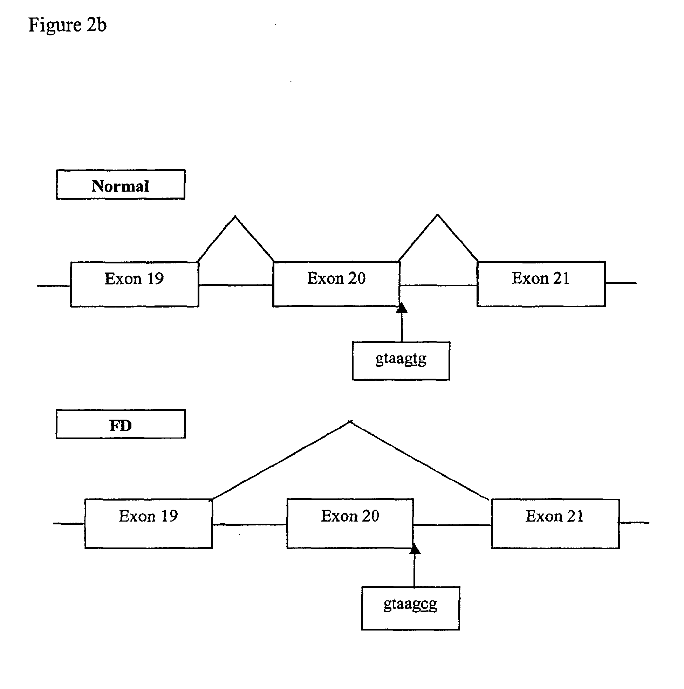

Sequence analysis of the IKAP-encoding gene reveals, in chromosomes with the major FD haplotype, a T → C transition in position 6 of the donor splice site of intron 20 (Fig. 2A). This mutation (2507+6T→C) results in the generation of an IKAP mRNA in which exon 20 is spliced out along with intron 20 (Fig. 2B).

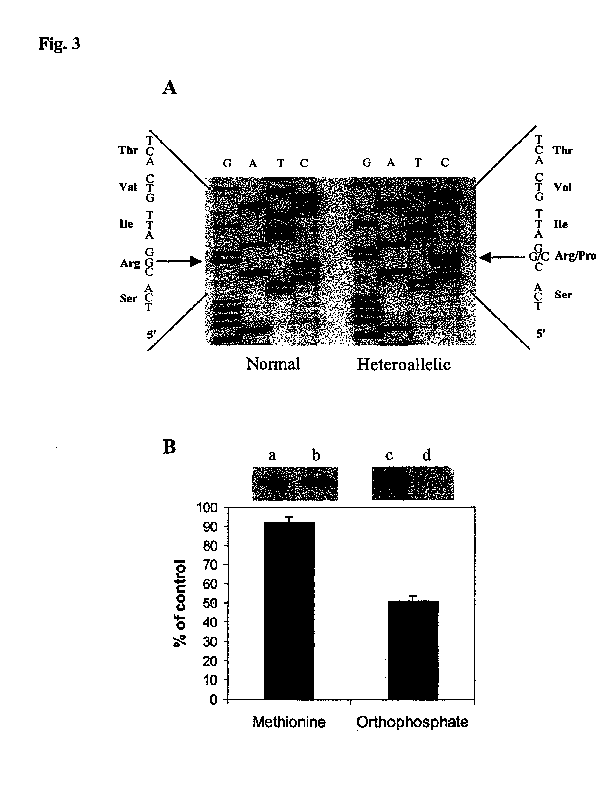

Characterization of the IKAP-encoding gene of individuals heterozygous for the FD chromosome with the most common minor haplotype (minor 2) (*8*) reveals, in this allele, a G → C transversion of nucleotide 2390 in exon 19 of the reported IKAP cDNA (*9*; Genbank accession #NM_003640), resulting in an arginine → proline substitution of amino acid residue 696 of IKAP (R696P) (Fig. 3A) and the disruption of a consensus serine/threonine kinase phosphorylation site (RIVT→pIVT). Thus, probands with both the 2507+6T→C and R696P mutations produce a truncated IKAP as well as a full-length IKAP with a non-consensus phosphorylation site, respectively.

The present invention, thus, relates in a first aspect to the identification of the gene responsible for FD, and the pertinent mutations and altered proteins associated therewith.

The present invention relates in a second aspect to a method for detecting the presence in a subject of a polymorphism linked to a gene associated with familial dysautonomia, said method comprising detecting a disruptive mutation in a gene of said subject encoding the IκB kinase-complex-associated protein.

The present invention relates in a third aspect to novel primers useful in the diagnostic methods according to the teachings of the present invention.

The accompanying drawings, which illustrate various aspects of the present invention, can be summarized as follows:
**Fig. 1.** A deletion in IKAP mRNA results in a truncated protein. **(A)** RT-PCR analysis of IKAP RNA. RT-PCR analysis was performed on RNA isolated from lymphoblast cell lines generated from individuals lacking the *DYS* allele (normal), or heterozygous (carrier) or homozygous (affected) for the major *DYS* allele. DNase-treated total RNA was amplified with primers spanning exons 19-21 (5'-GCAGCAATCATGTGTCCCA-3' (SEQ ID NO: 1) and 5'-GATTCTCAGCTTTCTCATGC-3' (SEQ ID NO: 2)). Arrows indicate the positions of the DNA markers run on the gel. **(B)** Alignment of normal and FD IKAP cDNA and amino acid sequences. An alignment of a portion of exons 19-21 of normal IKAP cDNA with that of IKAP cDNA from an FD-affected individual demonstrating that the exclusion of exon 20 in the RNA transcribed from the FD allele results in a frame shift, causing premature termination of translation and a protein truncated by 619 amino acids. Normal IKAP has 1332 amino acids. **(C)** Western blot analysis of IKAP protein in a non-FD lymphoblast cell line (DY491) and a lymphoblast cell line established from an FD-affected individual (GM05106). The blot was probed with polyclonal antibody to IKAP prepared in goat against the 18 aa peptide, DPVSREVKNEVSLVAEGF (SEQ ID NO: 3), encoded in exons 2 and 3. The presence of IKAP was detected with an anti-goat antibody conjugated to alkaline phosphatase. A prestained molecular weight marker was used to determine sizes of products (not shown).
**Fig. 2.** T→C transition in a donor splice site results in excision of exon 20. **(A)** Sequence analysis of IKAP intron 20 donor splice sites in normal and FD alleles. PCR fragments, amplified from DNA derived from normal cells and cells homozygous for the major FD haplotype, spanning the intron 20 donor splice of IKAP were sequenced. The normal donor splice sequence is GTAAGTG (SEQ ID NO: 4), the FD sequence is GTAAGCG (SEQ ID NO: 5). **(B)** Splicing of the IKAP transcript. Normal splicing of the IKAP transcript results in removal of introns 19 and 20 and retention of exon 20. The T→C transition in the donor splice site of intron 20 in the mutant allele results in removal of introns 19 and 20, as well as exon 20. The base change in the donor splice site is underlined.
**Fig. 3**. A G→C transversion prevents phosphorylation. **(A)** Sequence analysis of exon 19 of IKAP cDNA. cDNA from a normal individual and a carrier of the minor 2 haplotype was sequenced from PCR fragments spanning exon 19. **(B)** Immunoprecipitation of ³⁵S-methionine labeled versus ³²P-orthophosphate labeled IKAP. DY491, a normal lymphoblast cell line, and DY374, a lymphoblast cell line established from a carrier of the minor 2 FD haplotype, were labeled for six hours with ³⁵S-methionine or ³²P-orthophosphate. Whole cell extracts were prepared from these cells and equal amounts of acid-precipitable radioactivity were immunoprecipitated with an IKAP polyclonal antibody (described in Fig. 1C). The levels of radiolabeled IKAP protein precipitated from ³⁵S-methionine and ³²P-orthophosphate labeled DY374 cells, respectively, are depicted, in the bar graph, as a percentage of the radiolabeled protein precipitated from DY491 cells. The standard deviation for four experiments is indicated. Above the bars is an autoradiograph depicting the immunoprecipitated ³⁵S -labeled IKAP from DY491 (a) and DY374 (b) cells and the immunoprecipitated ³²P-labeled IKAP from DY491 (c) and DY374 (d) cells. Immunoprecipitations of IKAP from three other non-FD cell lines yielded results similar to that observed with the DY491 cell line (data not shown).

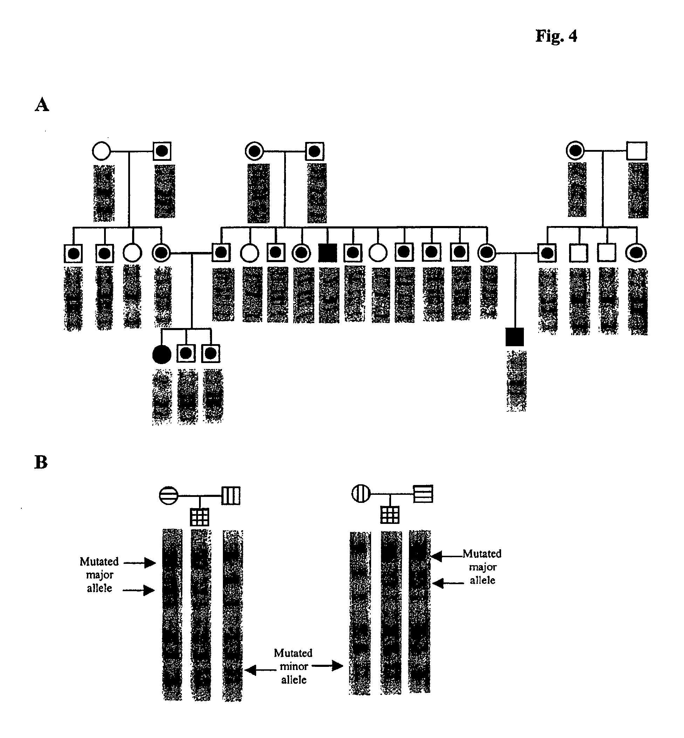

**Fig. 4.** Genotype analysis, using SSCP, of FD alleles. **(A)** PCR of the FD major allele in an extended family. A 244 bp fragment was amplified from DNA purified from blood using primers 5'-GAGAACAACAAGATTCTGC-3' (SEQ ID NO: 6) and 5'-AGTCGCAAACAGTACAATGG-3' (SEQ ID NO: 7) in the presence of α-³³P-dATP. The amplified products were denatured and fractionated on a 5% acrylamide non-denaturing gel at 4°C. Open circles (females) or squares (males) represent carriers of normal alleles, circles or squares with black circles inside represent heterozygous carriers of the major FD haplotype and filled circles and squares, FD-affected homozygous individuals with two mutated alleles. **(B)** Multiplex PCR of FD major and FD minor 2 alleles in two families. PCR was performed as in panel A, except two additional primers, 5'-GCAGTTAATGGAGAGTGGCT-3' (SEQ ID NO: 8) and 5'-ATGCTTGGTACTTGGCTG-3' (SEQ ID NO: 9), which generate a 238 bp DNA fragment, were included in the reactions. PCR reactions were analyzed as above. Parental carriers of the major or minor 2 allele are shown as circles (female) or squares (male) with horizontal lines or vertical lines, respectively. FD-affected offspring with both major and minor 2 alleles are shown as crosshatched.

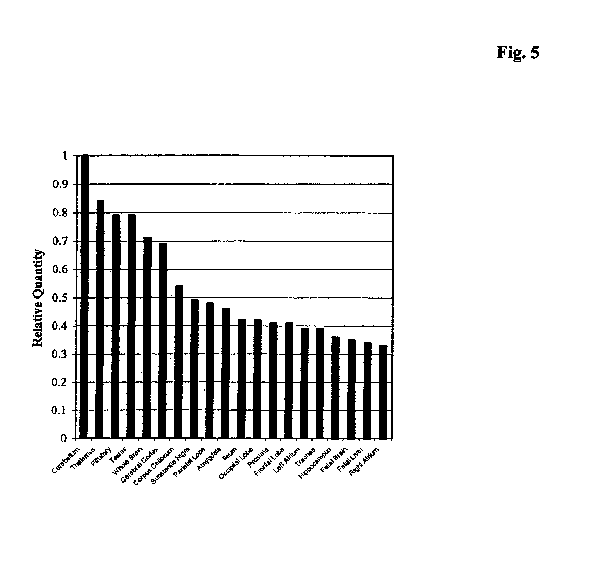

**Fig. 5.** Differential expression of IKAP by tissue type. A Multiple Tissue Expression Array (Clontech) containing RNA from 76 different human tissues and developmental stages was probed with a radiolabeled 556 bp cDNA fragment spanning exons 23-27 of IKAP. The probed array was subjected to autoradiography and densitometric scanning to quantitate relative levels of tissue expression. The 20 tissues that showed the highest level of expression are depicted. The highest level of expression was observed in the cerebellum, whose level was set at 1.0; the relative expression levels in the other 19 tissues are shown. The amounts of poly A+ RNA in the tissue samples on the array have been normalized based on eight housekeeping genes.

Immunoprecipitation of IKAP from ³⁵S-methionine or ³²P-orthophosphate-labeled cells derived from a normal individual and an individual heterozygous for R696P revealed comparable levels of synthesis of IKAP but a reduced level of phosphorylation of this protein in cells bearing the R696P mutation (Fig 3B).

SSCP analysis capable of differentiating between the normal and the mutated sequences of IKAP can be used to diagnose FD. The technique of SSCP analysis is well known. We started with a published protocol *(PCR Primer: A Laboratory Manual.* 1995. C.W. Dieffenbach and G.S. Dveksler, eds. Cold Spring Harbor Laboratory Press. Pages 249-255), but modified it to work for our test. To do SSCP in a clinical setting, DNA is isolated from the blood or tissue of subjects to be tested. The SSCP analysis can then be done using the following or other conditions:

PCR: 1-10 ng of DNA amplified in the presence of radioisotope (³³P-dATP); initial denaturation for 5 min at 94°C, followed by 50 cycles of 94°C x 30 sec, 58°C x 30 sec, 72°C x 30 sec; final extension at 72°C x 7 min.

Denaturation prior to running PCR samples on gel: PCR products diluted 1:6 in denaturation buffer. Final concentration of buffer components: 47.5% formamide, 13.3 mM EDTA, 0.033% SDS, 0.025% bromophenol blue, 0.025% xylene cyanol. Denaturation at 95°C x 5 min, followed by quick-cooling on ice.

SSCP gel: 1.5 µL of denatured PCR product loaded on 0.35 mm thick 5% nondenaturing acrylamide gel; run at 4°C x 3.75 hr @ 1100 volts.

For the PCR, we used the primers indicated above in the description of Fig. 4. However, primer design can be varied significantly, and we contemplate the use of any primer sets capable of amplifying the gene regions under investigation. The primers indicated above in the description of Fig. 4 are the ones we used to do the genetic testing and therefore are the best ones to our present knowledge. However, if the primers were shortened or lengthened by one or even a few bases, the amplification should still work. Most PCR primers work best when they're 18-25 bases long, have about 50% G/C content and do not have self-complementary regions. Other sequences in the DNA nearby could probably also be used to design primers, keeping the above requirements in mind.

SSCP analysis was performed on a multigenerational family with several FD-affected individuals bearing the major FD haplotype (Fig. 4A) and two pairs of apparently unrelated parents with probands that have alleles corresponding to both the major and minor 2 FD haplotypes (Fig. 4B). In the family with probands homozygous for the major haplotype, all of the affected individuals were homoallelic for 2507+6T→C and all of the parents were heterozygous. In the families with probands heterozygous for the major and minor 2 FD haplotypes, for both pairs of parents, one parent and the proband are heterozygous for the R696P and the other parent and the proband are heterozygous for 2507+6T→C (Fig. 4B).

Analysis of 31 probands homozygous for the major FD haplotype revealed that 100% of the probands were homozygous for 2507+6T→C, 100% of the parents were heterozygous for this mutation and four siblings of the probands had FD and were homozygous for the FD haplotype and the 2507+6T→C mutation. No other families with probands with the minor 2 FD haplotype were available for analysis. Study of a random group of 619 individuals of Ashkenazic Jewish descent revealed the presence of 25 carriers of 2507+6T→C and two individuals with R696P. This observed FD carrier frequency of approximately 1 in 23 is slightly higher than the reported frequency (*7*) and may reflect either an under-estimation based on the birth frequency or the nature or size of the sample characterized in this study. Each of the individuals with the 2507+6T→C and R696P mutations was found to have the polymorphic DNA markers associated with the FD major and minor haplotypes, respectively (*8*).

Characterization of the expression of the IKAP mRNA in multiple tissues revealed the greatest level of expression in the cerebellum, thalamus, pituitary and testes and significant expression in several regions of the brain (Fig. 5). Little IKAP mRNA was detected in colon, lung and ovary (data not shown). Many of the neurologic disorders observed in FD-affected individuals have been attributed to incomplete development of sensory and autonomic neurons (*1-3*). The high level of expression of IKAP mRNA in nervous tissues and the defective synthesis of this protein in individuals with FD suggest that the absence of normal IKAP may play a role in the observed abnormal intrauterine development and postnatal maintenance of neurons (*3*). Furthermore, the abundant expression of IKAP mRNA in the cerebellum and thalamus suggest that the disturbances of gait and coordination, as well as inappropriate perception of pain and temperature, might be due in part to dysfunctions in these brain loci, respectively.

IKAP was initially identified and named based on its reported ability to bind the IκB kinases (IKKs), the NF-κB inhibitory subunit IκB-α, NF-κB and the NF-κB-inducing kinase (NIK) and assemble these proteins into an active kinase complex (9). Recent studies, however, suggest that IKAP is not associated with the IKKs and plays no specific role in cytokine-induced NF-κB activation (*10*). Characterization of the amino acid sequence of IKAP reveals significant amino acid sequence homology with the *Saccharomyces cerevisiae* IKI3 (*11*) and ELP1 (*12*) proteins as well as similar proteins in *Schizosaccharomyces pombe* and *Arabidopsis thaliana*. The IKI3 gene product mediates, by a yet to be determined mechanism, sensitivity to the yeast killer toxin (*11*). ELP1 is a subunit of a multisubunit complex that is associated with RNA polymerase II and is required for the activation and transcriptional elongation of a large number of genes (*12*). If IKAP, like ELP1, is a part of the RNA polymerase II elongation complex and plays a role in gene activation, the absence of functional IKAP in FD-affected individuals may prevent gene activation events necessary for normal neuronal development and function.

Although an embodiment has been described using SSCP analysis, we contemplate the use of any analytical method capable of detecting the aforementioned mutations. Another way would be using ARMS-PCR (amplification refractory mutation system). Detecting the mutations by ARMS-PCR will ultimately be easier and less expensive than SSCP analysis. ARMS-PCR is used to detect allele-specific mutations by using two primers, a first one that recognizes a normal region of the allele and a second one that contains the mutated nucleotide at or near the 3' end of the primer, thereby allowing only amplification of the mutant allele. Designing suitable primers requires trial and error; also, the primer design should be such that four primers will work together in one PCR reaction to detect either one of the two mutations at the same time. The presence of amplified DNA would indicate the presence of a mutant allele in an individual and the size of the amplified product would indicate which of the mutations was present. These products are run on agarose gels at room temperature, thereby avoiding the use of the SSCP gels, which are much more trouble to run.

Identification of the mutations responsible for FD will enable the identification of carriers of this genetic disorder and may result in the development of effective therapeutic approaches for individuals with FD.

It should be understood that the preceding is merely a detailed description of one embodiment of this invention and that numerous changes to the disclosed embodiment can be made in accordance with the disclosure herein without departing from the spirit or scope of the invention. The preceding description, therefore, is not meant to limit the scope of the invention. Rather, the scope of the invention is to be determined only by the appended claims and their equivalents.

### References Cited

The references cited in the foregoing description are as follows:
1. C. M. Riley, R. L. Day, D. Greely, W. S. Langford, *Pediatrics* **3,** 468 (1949).
2. F. B. Axelrod, R. Nachtigal, J. Dancis, *Adv. Pediatr.* **21,** 75 (1974).
3. F.B. Axelrod, in *Primer on the autonomic nervous system,* D. Robertson, P. A. Low, R. J. Polinsky, Eds. (Academic Press, San Diego, 1996), pp. 242-249.
4. X. O. Breakefield *et al., Proc. Natl. Acad. Sci.* **81,** 4213 (1984).
5. X. O. Breakefield *et al., Mol. Biol. Med.* **3,** 483 (1986).
6. P. W. Brunt, V. A. McKusick, *Medicine* **49,** 343 (1970).
7. C. Maayan, E. Kaplan, S. Shachar, O. Peleg, S. Godfrey, *Clin. Genet.* **32,** 106 (1987).
8. A. Blumenfeld *et al., Am. J. Hum. Genet.* **64,** 1110 (1999).
9. L. Cohen, W. J. Henzel, P. A. Baeuerle, *Nature* **395,** 292 (1998).
10. D. Krappmann *et al., J. Biol. Chem.* **275,** 29779 (2000).
11. H. Yajima, M. Tokunaga, A. Nakayama-Murayama, F. Hishinuma, *Biosci. Biotechnol. Biochem.* 61, 704 (1997).
12. G. Otero *et al., Mol. Cell.* **3,** 109 (1999).

## Claims

1. A method for detecting the presence in a subject of a polymorphism linked to a gene associated with familial dysautonomia, said method comprising detecting a disruptive mutation in a gene of said subject encoding the IκB kinase-complex-associated protein.

2. The method according to claim 1, which comprises detecting a disruptive mutation in the gene encoding the IκB kinase-complex-associated protein which is present on chromosome 9q31.

3. The method according to claim 2, which comprises detecting a T → C change in position 6 of the donor splice site of intron 20 of the gene encoding the IκB kinase-complex-associated protein which is present on chromosome 9q31.

4. The method according to claim 2, which comprises detecting a G → C transversion of nucleotide 2390 in exon 19 of the gene encoding the IκB kinase-complex-associated protein which is present on chromosome 9q31.

5. The method according to claim 3 or 4, which comprises detecting said T → C change and/or said G → C transversion by single-strand conformational polymorphism (SSCP) analysis.

6. The method according to claim 5, wherein said SSCP analysis is carried out on a nucleic acid sequence amplified by polymerase chain reaction (PCR).

7. The method according to claim 6, wherein said nucleic acid sequence is amplified by PCR using one or more oligonucleotide primers selected from the group consisting of:
a) GAGAACAACAAGATTCTGC (SEQ ID NO: 6);
b) AGTCGCAAACAGTACAATGG (SEQ ID NO: 7);
c) GCAGTTAATGGAGAGTGGCT (SEQ ID NO: 8); and
d)

ATGCTTGGTACTTGGCTG (SEQ ID NO: 9).

8. An oligonucleotide primer selected from the group consisting of:
a) GAGAACAACAAGATTCTGC (SEQ ID NO: 6);
b) AGTCGCAAACAGTACAATGG (SEQ ID NO: 7);
c) GCAGTTAATGGAGAGTGGCT (SEQ ID NO: 8); and
d)


SEQUENCE LISTING

<110> Rubin, Berish
      Anderson, Sylvia

<120> Detection of Mutations in a Gene Encoding IKB Kinase-Complex
-Associated Protein to Diagnose Familial Dysautonomia

<130> JH/ml 000081ep

<160> 13

<170> PatentIn version 3.0

<210> 1
<211> 19
<212> DNA
<213> Homo sapiens

<400> 1
gcagcaatca tgtgtccca
   19


<210> 2
<211> 20
<212> DNA
<213> Homo sapiens

<400> 2
gattctcagc tttctcatgc
   20


<210> 3
<211> 18
<212> PRT
<213> Homo sapiens

<400> 3

Asp Pro Val Ser Arg Glu Val Lys Asn Glu Val Ser Leu Val Ala Glu
1               5                   10                  15

Gly Phe


<210> 4

<211>  7
<212>  DNA
<213>  Homo sapiens

<400>  4
gtaagtg
     7



<210>  5
<211>  7
<212>  DNA
<213>  Homo sapiens

<400>  5
gtaagcg
     7



<210>  6
<211>  19
<212>  DNA
<213>  Homo sapiens

<400>  6
gagaacaaca agattctgc
    19



<210>  7
<211>  20
<212>  DNA
<213>  Homo sapiens

<400>  7
agtcgcaaac agtacaatgg
    20



<210>  8
<211>  20
<212>  DNA
<213>  Homo sapiens

<400>  8
gcagttaatg gagagtggct
    20

<210>  9
<211>  18
<212>  DNA
<213>  Homo sapiens

<400>  9
atgcttggta cttggctg
   18



<210>  10
<211>  117
<212>  DNA
<213>  Homo sapiens

<400>  10
ccccaggaca caaagcttgt attacagatg ccaaggggaa acttagaagt tgttcatcat
   60

cgagccctgg ttttagctca gattcggaag tggttggaca aacttatgtt taaagag
  117



<210>  11
<211>  39
<212>  PRT
<213>  Homo sapiens

<400>  11

Pro Gln Asp Thr Lys Leu Val Leu Gln Met Pro Arg Gly Asn Leu Glu
1               5                   10                  15

Val Val His His Arg Ala Leu Val Leu Ala Gln Ile Arg Lys Trp Leu
            20                  25                  30

Asp Lys Leu Met Phe Lys Glu
        35

<210>  12
<211>  43
<212>  DNA
<213>  Homo sapiens

<400>  12
ccccaggaca caaagcttgt attacagact tatgtttaaa gag
   43

<210>  13
<211>  12
<212>  PRT
<213>  Homo sapiens

<400>  13

Pro Gln Asp Thr Lys Leu Val Leu Gln Thr Tyr Val
1               5                   10